Europäisches Patentamt

European Patent Office

Office européen des brevets

(11) Publication number: **0 148 644**
A2

# EUROPEAN PATENT APPLICATION

(21) Application number: 84309156.2

(22) Date of filing: 31.12.84

(51) Int. Cl.⁴: **C 12 N 5/00,** C 12 N 15/00, C 12 P 21/00, G 01 N 33/577, C 07 K 3/18, G 01 N 33/80 // A61K39/42 ,(C12P21/00, C12R1:91)

(30) Priority: 06.01.84 US 568739
26.04.84 US 604069

(43) Date of publication of application: 17.07.85
Bulletin 85/29

(84) Designated Contracting States: AT BE CH DE FR GB IT LI LU NL SE

(71) Applicant: **THE BOARD OF TRUSTEES OF THE LELAND STANFORD JUNIOR UNIVERSITY, Encina 105 Stanford University, Stanford, California 94305 (US)**
Applicant: **CETUS CORPORATION, 1400 Fifty-Third Street, Emeryville California 94608 (US)**

(72) Inventor: **Engleman, Edgar G., 60 Lane Place, Atherton California 94025 (US)**
Inventor: **Foung, Steven K.H., 148 Park Street, San francisco California 94110 (US)**
Inventor: **Grumet, F Carl, 825 San Francisco Court, Stanford California 94305 (US)**
Inventor: **Raubitschek, Andrew R., 475 Embarcadero, Palo Alto California 94301 (US)**
Inventor: **Larrick, James W., Star Route Box 48, Woodside California 94062 (US)**

(74) Representative: **Paget, Hugh Charles Edward et al, MEWBURN ELLIS & CO. 2/3 Cursitor Street, London EC4A 1BQ (GB)**

(54) A novel fusion partner and its products.

(57) By careful screening and mutation, a human-murine hybridoma suitable as a fusion partner for immortalizing an antibody-secreting B cell has been generated. The trioma fusion products of this immortalizing partner are stable producers of human monoclonal antibodies. A trioma which produces monoclonal human anti-varicella zoster is disclosed. A human anti-A IgM is also produced using this general method by the trioma S-H22 and by an alternate hydrid, HHAI.

## A NOVEL FUSION PARTNER AND ITS PRODUCTS

This invention was made with USA government support under HL 29572 awarded by the National Institutes of Health.  The Government has certain rights in this invention.

## Description

### Technical Field

The field of the present invention is the production of human monoclonal antibodies through fused hybrid cells and the antibodies produced.  In particular, the present invention relates to new monoclonal antibodies against varicella and human blood group A, to novel trioma producers of human monoclonal antibodies and to murine-human hybridoma immortalizing cell lines suitable for their preparation.

### Background Art

' Human antibodies have been used both for diagnostic and therapeutic purposes for a number of years.  Diagnostic techniques include blood typing for transfusion (Yankee, R.A., et al, New Eng. J. Med., 281:1208 (1969)); or transplantation (Grumet, F.C., et al, Hum. Immunol, 5:61 (1982)).

The surfaces of red blood cells contain large numbers of antigenic determinants that are classified into blood groups.  One well-known group of red blood cell surface antigens is the ABO group.  These antigens are used extensively in blood typing.  Most blood typing tests are based on hemagglutination and involve mixing a blood sample with a panel of typing reagents that react with various surface antigens and cause the cells to agglutinate. The presence or absence of agglutination is an indication of blood type. ABO typing reagents have traditionally been derived from whole human blood. Non-human blood type reagents, including murine monoclonal antibodies, have been reported. Voak, D., et al, Vox Sang (1980) 39:134-140 describes a murine monoclonal anti-A antibody. British patent application 209425 (published 3 November 1983) describes a murine monoclonal anti-B antibody.

Therapeutic applications include prophylaxis of Rh hemolytic disease, (Nusbacher, T., et al., New Eng. J. Med., 303: 935 (1980); and injection of anti-varicella zoster plasma (Grose, C., Human Herpes Virus Inf. (1982) Marcel Delcker, N.Y.). The oldest technique for obtaining these antibodies is isolation from immune serum. However, the small concentration of the antibody of desired specificity among those which are generally present in serum presents a serious drawback.

Recently, the production of human monoclonal antibodies has become possible and these may serve as tools in diagnostic testing and in therapy as outlined above. Two major approaches for the production of human monoclonal antibodies (Mab's) have been used: direct immortalization of immunized lymphocytes with Epstein-Barr Virus (EBV) and Mab production by hybridomas formed between immortalized human B cell lines (EBV), lymphoblastoid, or human or murine myelomas, and human B lymphocytes from an immunized host. Neither of these approaches has proved entirely satisfactory.

It is common experience among practitioners in the art that EBV transformation, while successful in forming Mab-secreting cultures, will often fail to provide antigen-specific EBV transformed cells which have sufficiently long life spans to provide reliable sources of the desired antibodies (Kozbor, D., et al, Hybridoma, 1: 323 (1982)). Thus, this method fails to provide reliably for antibody production over extended periods. Previously produced hybridomas between immunized human B cells and appropriately drug marked mouse or human myeloma or human lymphoblastoid cell lines have suffered from low frequency of hybrid formation in the case of human-human hybridizations (Olsson, L., et al, Proc Nat Acad Sci, 212: 767 (1980)) or chromosomal instability in the case of murine-human hybridomas (Nowinski, R., et al, Science 210: 537 (1980), Lane, H. C., et al, J Exp Med, 155: 333 (1982)). (Murine-murine hybridomas are stable, but the antibodies produced are immunogenic if used in passive therapy.)

An immunized experimental animal can sometimes serve as a source for specific antibody-secreting B cells to provide the immunized lymphoid member of the hybridoma. This method cannot be used, however, to provide reagents for HLA or other blood type testing since when human antigens are injected, the plethora of antibodies elicited is mostly immunoreactive to antigens common to all humans, and the desired antigen-specific antibody is formed only as a very small percentage of the total response. Further, these non-human antibodies can themselves result in an adverse immune response if injected for human therapy.

The problems associated with human monoclonal antibody production have been greatly ameliorated by the immortalizing hybridoma and resultant triomas of the present invention. The present invention, thus, provides a means for producing human antibodies of the desired specificity dependably and over long periods of time, using a stable, immortal Mab producing source.

-4-

Disclosure of the Invention

The invention, in one important aspect, provides an ideal fusion partner for specific B-lymphoid cell lines or spleen cell lines, producing triomas that secrete specific antibodies of human character.

These "immortalizing" hybridomas of the invention are extraordinarily stable and retain sufficient human character that when fused with human spleen cells or immunized human lymphoid cells, form triomas which have the capacity to secrete human antibody. The monoclonal antibodies produced are of human character and should be of low immunogenicity when used in therapeutic applications. Furthermore, the immortalizing hybridoma also appears to confer stability on the resulting daughter hybrid with respect to its ability to produce the desired antibody.

Thus, this aspect of the invention concerns an immortalizing, non-secreting hybridoma having human characteristics. It is prepared by fusing mouse myeloma cells with human B lymphocytes and selecting the fusion product for stable immunoglobulin secretion and HLA surface antigen production, followed by treating the selected fusion product with mutagen and selecting the mutated product for non-secretion of immunoglobulin but retention of HLA antigen production. A particular embodiment of such an immortalizing hybridoma has been designated SBC-H20 and deposited at the ATCC on or about December 13, 1983, and has the ATCC # HB 8464.

In a second aspect, the present invention relates to the products of fusing the immortalizing hybridomas with suitable human immunized lymphoid cells. These "triomas" have the enhanced stability above referred to, and are useful sources of desired Mabs.

One specific embodiment of such triomas secretes anti-varicella zoster virus antibody. This trioma, designated SBC-H21, was deposited on or about December 13, 1983, and given the ATCC # HB 8463. Another specific embodiment secretes human anti-blood group A Mab, was designated S-H22, and deposited with ATCC March 1, 1984 and given accession # HB 8518. The invention also relates to methods of producing such triomas and of utilizing them for antibody production. Additional cell lines producing the Mabs of the invention are obtained from an alternate hyperdiploid fusion partner, LT228. One line, also a part of the invention, was designated HHA1 and deposited with ATCC March 28, 1984 as accession # HB 8534.

In still other aspects, the invention relates to the human monoclonal anti-A and anti-varicella antibodies which are produced by the triomas of the invention and to their diagnostic and therapeutic compositions and uses.

Modes of Carrying Out the Invention

A. Definitions

As used herein, "trioma" refers to a cell line which contains genetic components originating in three originally separate cell linages. As used in the context of the invention, these triomas are stable, immortalized antibody producers which result from the fusion of a human-murine hybridoma with a human antibody producing B cell.

The human-murine hybridoma (the "immortalizing hybridoma") is an immortal cell line which results from the fusion of a murine myeloma or other murine tumor cell with human lymphoids derived from a normal (preferably non-immunized) subject. As described in detail below, by careful selection and mutation, an immortalizing hybridoma which provides improved chromosomal stability, has human characteristics, and which does not secrete immunoglobulin is obtained. The antibody secreting capacity of the trioma is provided by the third member of the fusion which is typically derived either from B cells of an immunized human individual, or with such B cells altered so that they, too, are immortal.

"Non-secreting" hybridoma refers to a hybridoma which is capable of continuous reproduction and, therefore, is immortal, which lacks the capacity to secrete immunoglobulin.

A hybridoma "having human characteristics" refers to a hybridoma which retains detectable human-derived chromosomes such as those producing human HLA antigen which will be expressed on the cell surface.

Lymphoid cells "immunized against a predefined determinant" refers to lymph cells derived from an individual who has been exposed to an antigen having the determinant of choice. Thus, for example, an individual can be induced to produce from its lymphoid B cells antibodies against the antigenic determinants of various blood types, by virtue of exposure, through transfusions or previous pregnancy, or against the antigenic determinants of specific viruses or of bacteria by virtue of exposure through past infections or vaccinations. B cells which produce such antibodies are defined by this term.

"Functional equivalent" is intended to mean a human monoclonal antibody that recognizes the same antigenic determinant and cross-blocks the specific exemplified human immunoglobulin referenced, e.g. the varicella zoster Mab or anti-A Mab produced herein.

"Cell line" refers to various embodiments including but not limited to individual cells, harvested cells and cultures containing cells so long as these are derived from cells of the cell line referred to. By "derived" is meant progeny or issue. It is, further, known in the art that spontaneous or induced changes can take place in karyotype during storage or transfer. Therefore, cells derived from the cell line referred to may not be precisely identical to the ancestral cells or cultures, and any cell line referred to includes such variants.

As used herein the terms "blood group A" and "type A" are synonymous and are intended to include all red blood cells having one or more determinants found solely on group A blood cells (i.e., all A and AB groupings).

## B. General Method

The non-secreting, characteristically human hybridomas of the present invention are prepared by fusing murine myeloma cells with human peripheral B lymphocytes derived from peripheral blood, spleen or lymph nodes of human donors, followed by careful selection for clones and by mutagenesis. The resultants are suitable for subsequent fusion with antibody-producing lymphocytes. Fusions are carried out using standard conditions known in the art, such as, for example, incubation in the presence of polyethylene glycol, followed by selection for the desired fusion products. In the context of the present invention, the selection process is important, particularly with regard to the immortalizing hybridomas which must be carefully cloned by limiting

-8-

dilution and screened to assure the recovery of a cell line with the desired characteristics.

## B.1. Component Cell Lines

### B.1.a. For Immortalizing Hybridomas of the Invention.

Fusion partners which make up the immortalizing hybridomas are murine myeloma cells and human lymphoid B cells. Murine myeloma cell lines are commonly available and may be obtained through the ATCC. Human lymphoid B cells are isolated from the plasma of normal individuals using conventional techniques. Such procedures include density gradient purification and separation of B cells from T cells using standard sheep erythrocyte rosetting techniques known in the art.

### B.1.b. For Mab-Producing Cell Lines.

The Mab-producing cell line may be a trioma produced from the hybridoma of the present invention, preparation of which is described in detail hereinbelow. The antibody-producing component can be prepared using standard techniques from a choice of sources dependent on the antigen which the Mab is desired to recognize. Where an immunocomplexing reaction against a determinant associated with an infectious agent such as virus or bacterium is desired, plasma from an individual having had an infection with this agent is one source of these antibodies. Animal sources may also be produced in this case after deliberate innoculation to produce infections. Such non-human origins are not as desirable for therapeutic applications since the resulting antibodies, though produced by the method of the invention, retain non-human character and therefore may be somewhat immunogenic. For diagnostic applications, however, this is not a serious drawback.

When it is required to obtain antibodies reacting against normal human antigens such as, for example, the blood type antigens A or B or the characteristic HLA antigens exhibited by particular individuals, normal donors having the appropriate antibodies by virtue of transfusion or pregnancy are required. Ordinarily, the source of such cells would be peripheral blood; however, if available, spleen cells or lymph node cells are a second source of these antibody-producing cells. Separation and isolation techniques are described above.

The antibody producer may also be prepared by in vivo transformation of the appropriate B-lymphocytes with an infectious virus so that they are immortalized. Typical of such techniques is that described for transformation with Epstein-Barr virus (EBV) by Sly, W. S., et al, Tissue Antigens, 7:165 (1976). Still another alternative is use of a human derived immortalizing partner such as LT228 illustrated below.

While the monoclonal antibodies that are specifically exemplified herein are a human IgM which is an anti-red blood cell and a human IgG1 which is anti-varicella zoster, the invention is not limited to any particular species, class, or subclass of immunoglobulin. Other specific monoclonal antibodies may be other members of the IgG subclasses such as IgG1, IgG2, IgG3, etc., or other members of the IgM class. The members of the remaining immunoglobulin classes IgA, IgE or IgD can also be produced by trioma fusion products of the murine-human, non-secreting hybridoma and identified by following the screening procedures described herein.

-10-

B.2. <u>Fusion Procedure</u>.

Fusions to form the murine-human non-secreting hybridomas and the triomas of the inventions as exemplified are performed basically by the method of Kohler and Milstein, <u>Nature</u>, 256:495 (1975). Briefly, a tumor cell line (to make the hybridomas) or hybridoma (to make the triomas) is combined with partner cells (typically spleen cells or B lymphocytes) that produce the antibody of interest using a fusogen such as polyethylene glycol under suitable conditions-- typically 40%-50% polyethylene glycol, of MW 1000 to 4000 at room temperature to 40°, preferably about 37°. Fusion requires about 5-10 minutes, and the cells are then centrifuged and screened.

B.3. <u>Screening Procedure</u>.

After any fusion procedure, screening for cells which are, indeed, hybridized products is made by culturing cells centrifuged from the fusion medium in growth medium which is selective for the desired hybrids. Ordinarily, non-immortalized fusion partners cannot survive repeated transfers on any medium, and hence will not survive repeated culturing of the centrifuged cells. Commonly used lines of immortalized murine myeloma cells, however, are incapable of growth on certain selective media which have been chosen to deprive them of their ability to synthesize DNA. Two very commonly used media of this description are "hypoxanthine-aminopterin-thymidine" or "HAT" medium and azaserine-hypoxanthine medium or "AH" medium. Both of these selection media take advantage of the capacity of the normal cells to utilize a "salvage" pathway for DNA synthesis under circumstances where the <u>de novo</u> process is inhibited. This salvage process, which requires hypoxanthine phosphoribosyl transferase (HPRT) is generally inoperable in these commonly used murine myeloma cells (although they retain the de novo pathway). Thus, both normal cells and the hybrids (which acquire the normal cells' ability to synthesize HPRT) can grow in medium containing hypoxanthine even if the de novo synthesis of DNA is inhibited by the medium; tumor cells cannot. Aminopterin in the HAT medium, and azaserine in the AH medium are both inhibitors of the de novo DNA synthesis pathway. (Aminopterin inhibits both purine and pyrimidine nucleotide synthesis and thus thymidine is required as well as hypoxanthine for the salvage pathway. Azaserine inhibits only purine synthesis, so only hypoxanthine for the salvage pathway is required.)

In short, only hybridized cells can both survive repeated transfers and grow in HAT or AH medium--normal cells cannot survive because they are not immortalized and do not survive repeated transfers; unhybridized tumor cells cannot survive because they lack the salvage pathway which permits the use of hypoxanthine to overcome ammopterin or azaserine inhibition.

Successful fusions are first produced by using AH or HAT medium, but additional selection procedures are required. This is accomplished in part by limiting dilution and individual clone analysis.

In those special instances where the immortalized hybridoma is to be fused to a transformed antibody producer, an additional property is required. The transformed antibody producer does not die from multiple transfers as would a normal cell and unlike the common murine myeloma immortalizing lines is not sensitive to HAT or AH medium. Thus, these usual selection means will permit such unfused, transformed lymphocytes to survive. A screening procedure for successful triomas thus requires inclusion in the medium of a drug to which these transformed parent cells are sensitive. Therefore, the immortalizing hybridoma must, in addition to other desired properties, have acquired resistance to this drug so that it can transfer resistance to the trioma. In the example below, the drug is ouabain.

As mentioned above, the selection procedure employed in selecting the immortalizing hybridomas of the invention goes far beyond simple selection for successfully hybridized murine-human hybridomas. Additional properties in the cell line, are required and must be selected for or imparted by mutagenesis. These immortalizing hybridomas must show stable human characteristics, non-secretion of immunoglobulin, sensitivity to a medium to which the fusion partner will be resistant, and if an immortalized lymphoid partner is used, resistance to a drug capable of destroying the fusion partner. This particular collection of characteristics requires a unique and well-designed screening and mutagenesis process.

Briefly, the cells centrifuged from the fusion mixture are diluted and plated in microtiter plates or other convenient means for growing large numbers of single colonies. Screening is done using AH or HAT medium growth. Selection from successful colonies is made on the basis of assay procedures related to stability and human character. Thus, from among the many colonies assayed, several are chosen which continue to produce immunoglobulin in the supernatant fluid for a suitable period of time, preferably in excess of six months (one criterion for stability). The continued production of such immunoglobulin indicates that the characteristics conferred by the lymphocyte partner have not been lost (lymphocytes which were unfused will, of course, not survive). Retention of human characteristics was assessed by assaying the cell surfaces for the presence of HLA antigen. The selected colonies continue to exhibit HLA antigen production at their cell surfaces (another indication of stability, as well as human character).

The selected clones, are then subjected to a mutagen such as 6-thioguanine in order to destroy their ability to secrete immunoglobulin and confer HAT or AH sensitivity, and ouabain resistance. This will clear the way for later fusion to give a trioma, and subsequent use of the fusion product to secrete a particular monoclonal antibody characteristic of the added fusion partner. Accordingly, the selected cell lines are grown in the presence of a mutagen such as 6-thioguanine in increasing concentrations, and mutants selected for inability further to secrete immunoglobulin, HAT/AH sensitivity and ouabain resistance. However, only those colonies which remain capable of surface HLA antigen expression are chosen.

In summary, the immortalizing hybridoma which is central to the invention herein, is derived on the basis of a very careful selection/mutagenesis procedure from its human-murine parents. The initial selection is based on HAT/AH sensitivity, Ig secretion, and HLA surface antigen production. Mutation causes loss of endogenous Ig secretion and restores AH/HAT sensitivity, but HLA production is retained.

B.4. <u>Production and Purification of Human Monoclonal Antibodies From Cultured Cells.</u>

After fusion with an antibody-producing human lymphoid cell in accordance with the procedures set forth above, the immortalizing hybridomas of the invention are converted to trioma producers of the specific antibody brought to the union by the new partner.

The resulting triomas are selected by using an appropriate selection medium. If the antibody-producing partner is a normal cell line, selection medium can simply be the HAT or AH medium which will discriminate against unfused immortalizing hybridoma cells--the antibody producer fails to maintain immortality in successive transfers. If the antibody producer is itself an immortalized cell--i.e. for example, a virus transformed lymphocyte, an additional selection in the presence of, for example, ouabain to kill these unhybridized cells is also required. Alternative selection procedures are, of course, possible depending on the nature of the cells used in the fusion. It would, for example, be possible to confer by mutagenesis alternate sensitivities on the immortalizing hybridoma which would respond to other medium selecting factors besides HAT and AH.

Clones having the required specificity are identified by assaying the trioma culture medium for the ability to bind to the desired antigen and the nature of the Mabs may be further characterized by testing for specificity. Triomas that produce human antibodies having the desired specificity may be subcloned by limiting dilution techniques and grown <u>in vitro</u> in culture medium or injected into selected host animals and grown <u>in vivo</u>.

The antibodies may be separated from resulting culture medium or body fluids by conventional antibody fractionation procedures such as ammonium sulfate precipitation, DEAE cellulose chromatography, affinity chromatrography and the like.

B.5. Use of Mab Produced By the Method of the Invention As A Diagnostic.

If desired, the human monoclonal antibodies may be derivatized (labeled) using conventional labeling reagents and procedures. As used herein, the term "label" is intended to include both moieties that may be detected directly, such as radioisotopes or fluorochromes, and reactive moieties that are detected indirectly via a reaction that forms a detectable product, such as enzymes that are reacted with substrate to form a product that may be detected spectrophotometrically.

The use of Mab in detecting a desired antigen is substantially an immunoassay, and a variety of conventional immunoassay procedures such as ELISA or RIA may be used. If the antigen is already immobilized on a solid insoluble support, the antibody may be applied to the support, incubated under conditions that allow immune complex formation between the antibody and any immobilized antigen on the support, and the support washed to removed unbound antibody. Temperature, pH, and duration are the most important conditions in the incubation. The temperature will usually range between 5°C and 40°C, the pH will usually range between 6 and 9 and the binding reaction will usually reach equilibrium in about 1 to 18 hr. Antibody will normally be used in excess.

In instances where the antibody is labeled directly, immune complexes may be detected via the label, eg, radioactive or fluorescent on the antibody. A more common and preferred procedure is to use unlabeled monoclonal antibody and incubate the desired antigen-monoclonal antibody complex with an enzyme-conjugated antibody against the monoclonal antibody. The same incubation conditions as were used in the initial incubation may be used. The resulting ternary complex may be treated with substrate and detected spectrophotometrically via an enzyme-substrate reaction. By using conventional procedures in which the detection means is bound indirectly to the antigen-monoclonal antibody via one or more layers of immunochemical, it may be possible to amplify the detection signal to improve the sensitivity or the detection limit of the procedure.

Kits for carrying out the above described tests will normally contain an antigen immobilizing material, the monoclonal antibody, enzyme-conjugated antibody against the monoclonal antibody and an appropriate substrate. The kits may also contain a suitable buffer for dilution and washing, a post-coating preparation such as bovine serum albumin and directions for carrying out the tests. These components may be packaged and stored in conventional manners.

B. 6. Use of the Mabs of the Invention in Purifying Antigen.

The antibodies may also be covalently coupled to chromatography supports (eg, the surfaces of tubes or plates or the surface of particulate bodies such as beads) using available bifunctional coupling agents, such as carbodiimides, to make effective adsorbents for affinity purifying a desired antigen. A solution of the antigen in cold buffer at pH 7-7.5 is passed through a column containing the monoclonal antibody fixed to a support. The desired antigen will be retained by the column and may be eluted therefrom with an appropriate elutant.

Kits for isolating the desired antigen will contain the monoclonal antibody conjugated to the support, buffer to serve as a medium for adsorbing the antigen to the support and an elution reagent.

B.7. Use of the Mabs of the Invention in Therapy.

The use of Mab's in therapy has been limited due to the difficulty of providing a stable source of human monoclonal antibodies. However, the method based on the invention is similar to that presently employed using polyclonal sources. A particularly well known application is the use of gamma globulin to prevent infection. References to the use of immune plasma in immunocompromised patients for prevention of varicella-zoster infection, and for prevention of Rh hemolytic disease in the newborn have been cited above. Briefly, the antibody is injected in a plasma-compatible solution in amounts determined by the needs of the subject.

C. Examples

The following examples serve to illustrate the invention and are not intended to limit its scope. While the fusion partners for combination with the immortalizing hybridoma of the invention have led to the production of human anti-red blood cell type A and human anti-varicella monoclonal antibodies, by a suitable alternate selection of the secreting B cell, a variety of monoclonal antibodies may be produced, such as, for instance, antibodies against E. coli, cytomegalovirus (CMV), hepatitis (HBV) or other infective agents and against red blood cell type B and against HLA antigens. Further, in producing the immortalizing hybridoma, B cells from a normal particular individual have been used, of course, any normal B lymphocytes can be utilized as the fusion partner with mouse myeloma.

C.1.  Preparation of SBC-H20; An Immortalizing Hybridoma.

Mouse myeloma cell line SP2/08A2 was obtained for use as the immortalizing partner from Frank Fitch, University of Chicago. This cell line is freely available and can be used without restriction. Other mouse myeloma lines are also readily available. Human peripheral B lymphocytes were isolated from the heparinized plasma of a normal human donor by Ficoll-Hypaque gradient as described by Boyum, A., Scand J Lab Clin Invent 21: (Suppl. 97) 77 (1968). The peripheral B lymphocytes B and myeloma cells were mixed at a 1:1 ratio, washed once in RPMI 1640 medium (Gibco), and pelleted at 250 x g for 10 minutes. The pellet was gently resuspended in 1 ml of RPMI with 40-45% (volume/volume) polyethylene glycol solution, MW 1430-1570 (BDH Chemicals, Poole, England) which was prewarmed to 37°C. After two minutes at room temperature, the cell suspension was diluted to 6 ml with RPMI, centrifuged at 500 x g for 3 minutes, and, beginning 8 minutes from the onset of fusion, the cell pellet washed with RPMI containing 10% fetal calf serum. The pelleted cells were plated in multi-well trays using suitable dilutions to obtain individual clones. The colonies were grown on AH selection medium containing 2 μg/ml azaserine and 100 μM hypoxanthine, and successful clones were assayed for immunoglobulin production and for HLA surface proteins using the assay methods as set forth in paragraphs C.10.a and C.10.b herein.

A hybrid clone which had had a stable immunoglobulin production for 6 months, and which was consistently producing HLA surface protein was selected.

This clone was then placed in Iscove's medium (IDMEM) (Gibco) containing 10% fetal calf serum, 2 mM glutamine, 100 units penicillin, 100 mg streptomycin per ml, as well as the mutagen $2x10^{-6}M$ thioguanine (Sigma). The concentration of 6-thioguanine was progressively increased to $2x10^{-5}M$ over a period of approximately 30 days. The resulting mutant hybrids were sub-cloned, and the colonies tested for immunoglobulin secretion. A non-secreting sub-line which was HAT/AH sensitive, resistant to $10^{-6}$ M ouabain, and which retained ability to produce HLA surface antigen, was designated SBC-H20. A sample of this cell line was deposited with ATCC on December 13, 1983, and given the designation ATCC HB 8464. The characteristics of this murine-human hybridoma include: sensitivity to HAT and AH media, resistance to ouabain (Sigma) to a concentration of $10^{-6}$ M, non-secretion of immunoglobulins, human chromosomal stability over time, and production of HLA surface protein.

C.2.   Preparation of an Alternate Fusion Partner--
       Ouabain Resistant LTR228.

LTR228 is a subvariant of the WI-L2 line (Levy, J. A., et al, Cancer (1968) 22:517). It was derived from a mycoplasma-contaminated generic WI-L2 parent by cloning the parent in soft agar, decontaminating the parent line, and culturing it in Iscove's medium containing 20 µg/ml 6-thioguanine (6-TG). LTR228 was selected from among the 6-TG resistant clones on the basis of its ability to fuse efficiently with normal B lymphocytes to produce stable human x human hybridomas.

LTR228 has a hyperdiploid modal chromosome number of 8. LTR228 cells are characterized by having: extra copies of chromosome 13 and 20; a Robertsonian translocation between chromosomes 14 and 21; a copy of chromosome 8 with an enlarged short arm composed of a homogeneously staining region; and a marker 21 which has a translocation from the distal end of chromosome 11. LTR228's karyotype is: 48,XY,+13,+20,-14,+t(14q;21q),-21,+der(21),t(11;21) (q13;p11),8pt+. LTR228 secretes small amounts of IgM and has a doubling time of about 16 hr. Its rapid growth rate and high cloning efficiency both in soft agar and by limiting dilution are important characteristics of the line.

LTR228 is the subject of a concurrently filed U.S. patent application titled "Human Lymphoblastoid Cell Line and Hybridomas Derived Therefrom."

LTR228 cells were made resistant to ouabain by culturing them in ICM containing $10^{-8}$ ouabain. Resistant cells were expanded and the concentration of ouabain was increased gradually. The procedure was repeated until the cells could survive $10^{-6}$ M ouabain. Clones were selected from soft agar supplemented with 6-TG (20 µg/ml) and ouabain ($10^{-6}$ M).


C. 3. Preparation of Anti-A Secreting
EBV Transformed Lymphocytes.

Lymphocytes were isolated from a spleen removed from a type O individual (who required splenectomy because of a severe hemolytic anemia) by separation on a Ficoll-Hypaque density gradient of specific gravity 1.077 according to the procedure of Boyum, A. (supra). The cells were cultured at a cell concentration of $2 \times 10^6$/ml in IDMEM containing 15% fetal calf serum and 0.03% type Al red blood cells. After three days, T cells were removed by the single step rosetting method of Saxon, A., et al, J Immunol Methods, 12: 285 (1976) using 2-aminoethylesothiouronium bromide hydrobromide, and the residual B cells were transformed by the EBV containing supernatant from the marmoset line B-958 according to the procedure of Sly, et al (supra). The cells from the transforming mixture were transferred to microtiter plates at a concentration of $10^5$ cells per well and cultured for 14 days in IDMEM with 15% FCS medium.

Anti-A Ig-containing wells were identified by the type A red blood cell (ARBC) agglutination test described by Parker, J., et al, Transfusion (1978) 18:417. Two positive wells were identified, but only one, designated E6, survived. E6 cells were enriched by treatment with papain and rosetting with ARBC. Rosetting cells were separated by ficoll-hypaque gradient centrifugation (yield 10%) and then cloned in soft agar. Colonies were picked, expanded and assayed for anti-A activity by ARBC agglutination. One subclone, identified as E6C6, was chosen for fusion with the fusion partners described below.

Supernatant from E6C6 was further tested against random human donors' red blood cells of groups Al, A2, B, O, Aint, AlB, and A2B. The supernatant reacted with all A groups and no others. Supernatant was also tested against a panel of rare A subgroups (A3, Ax, Aend, A5(l)) together with two commercial antisera. The table below shows the results of this testing.

| Cell | Monoclonal Antibody | Commercial* | Commercial A,B |
|------|---------------------|-------------|----------------|
| A3 | 2 (M.F.) | 3 (M.F.) | 3 (M.F.) |
| Ax | +w | 1 | 2 |
| Aend | +w | +w | +w |
| A5(1) | 1 | 0 | 0 |

*.Obtained from Dade (A-A26-16, A,B-0151-2B)
0  = no reaction;
+w = weak, but definitely positive reactivity;
1-3 = increasing strength.
M.F.= mixed field reaction of positive reactivity;

This monoclonal antibody was also tested by a commercial blood banking laboratory on previously documented red cell phenotypes of groups A2, A1, A1B, A2B, B and O. The antibody agglutinated all A and AB phenotypes but did not agglutinate the B and O phenotypes.

The class of the antibody produced by E6C6 was determined by conventional isotyping procedures to be IgM with kappa light chain.

### C.4. Preparation of Anti-Human A1 Secreting Triomas from SBC-H20.

Triomas producing anti-human A antibodies were prepared using the fusion procedures set forth in paragraph C.1 except using a 1:10 mixture of the anti-A lymphocytes E6C6, to SBC-H20 cells (or SP2/08A2 cells as a control). After washing in RPMI with 10% fetal calf serum, the cells were plated in microtiter plates at a density of $1 \times 10^6$ cells per well over irradiated mouse spleen feeder cells and grown under a humidified 6% $CO_2$

atmosphere at 37°C. Two similar selection media were used; HAT selection medium containing 100 $\mu$M hypoxanthine, 50 nM aminopterin, 15 $\mu$M thymidine, as well as 0.1 mM ouabain and AH selection medium containing 2 $\mu$g/ml azaserine, 100 $\mu$M hypoxanthine and 0.1 $\mu$M ouabain. Unfused SBC-H20 and unfused EBV transformed anti-A E6C6 lymphocytes were dead after 10 days. The resulting fused cells were further selected for 10 days using 0.1 $\mu$M ouabain and for 14 days by HAT or AH (supra). Individual clones were verified by sequential soft agar and limiting dilution cloning.

C.5. Preparation of Anti-A Secreting Cells from LTR228.

The fusion mixture contained PEG 4000, 40% (w/v); dimethylsulfoxide (DMSO), 10% (v/v) in Hank's balanced salt solution (HBSS)-/+ ($Ca^{2+}$ free, 2 mM $MgSO_4$), supplemented with 5 $\mu$g/ml poly-L-arginine (Sigma, 70K-150K). Forty g of PEG 4000 were combined with 10 ml of DMSO and 50 ml of HBSS-/+. The mix was autoclaved for 25 min. When the solution had cooled to room temperature, poly-L-arginine from a filter sterilized 1000x stock solution was added to obtain a final concentration of 5 $\mu$g/ml. Before use, the pH of the fusion mixture was adjusted to 7.9 with sterile 0.1 N NaOH. Fresh fusion mixture was made every two to three weeks.

Plates (Costar 3506, 6-well cluster, 35 mm well diameter) were prepared as follows: 2 ml of HBSS-/+ and 50 $\mu$l of a filter sterilized, 100 $\mu$g/ml, peanut agglutinin (PNA, Sigma) were added to each well. Plates were incubated at 37°C for at least one hr prior to use. PNA stock was stored frozen, and a freshly thawed aliquot was used to coat fusion cells. Smaller sized wells were used if cell numbers were limited.

Parent cells were washed twice in HBSS-/+ at room temperature and subsequently resuspended and combined in HBSS-/+ warmed to 37°C. Two ml of the suspension (10-20 million cells) were added to each pretreated well containing PNA coating solution. Plates were spun at 400-500 x g, room temperature, for six min to form a monolayer of cells. Supernatant was then aspirated off the plates.

Two ml of fusion mixture warmed to 37°C were carefully added down the side of the fusion cell. After one min, the PEG solution was diluted with 37°C 5% DMSO in HBSS-/+ (filter sterilized) at a rate of 2 ml/min (0.5 ml every 15 sec) for three min (6 ml). The fusion dilution mixture (FDM) was then added at a rate of 4 ml/min until the well was filled. FDM was always added down the side of the well, so as not to disturb the monolayer, and the plates were constantly swirled to ensure optimal mixing.

At this stage, the wells were aspirated. The remaining film of PEG mixture was diluted at a rate of 2 ml/min for two min with warm FDM. Again the plate was constantly swirled. Over a period of 15 sec, 5 ml of 37°C HBSS-/+ were added to the fusion well, and all supernatant was aspirated from the monolayer. Finally, each fusion well was washed twice with 5-10 ml of warm HBSS-/+. Five ml of warm ICM, 15% FCS, were added to each well, and the plates were incubated at 37°C. The day following fusion the cells were plated at $10^5$ cells/well in ICM containing azaserine (2 µg/ml) and ouabain ($10^{-6}$ M). Growing hybrids were visible by day 10 and growing wells were tested for anti-A activity by hemagglutination on day 21. Positive wells were recloned in soft agar. A sample of one of the anti-A producing subclones, designated HHA1, was selected for preservation and deposit.

C.6. Verification of Trioma Anti-A Forming Clones By Chromosomal Analysis.

Chromosome preparations were obtained from the trioma clones and assessed to verify successful fusions.

Chromosome preparations were made as follows: Approximately $10^6$ cells per ml were placed in basic growth medium containing vinblastine at 0.5 g per ml (Lilly), incubated for 3 hours, and centrifuged at 250 x g for 10 minutes. The cell pellet was resuspended in 5 ml of hypotonic solution (growth medium/distilled water, 1:4) for 10 minutes and the cells repelleted, washed twice in fixative comprising methanol:glacial acetic acid, 5:1, and finally resuspended in a few drops of fixative. The suspensions were air dried on microscope slides, stained with Giemsa (Sigma) and examined microscopically.

The triomas were thus shown to contain 100-110 chromosomes, of which 70-75 have acrocentric centromeres suggestive of murine origin, and the rest of which have metacentric centromeres, suggestive of human origin. (The parent SBC-H20 cells have 75-80 chromosomes; 3 with metracentric centromeres.)

C.7. Anti-Human Al Antibody Production by Trioma SBC-H20 x E6C6.

Supernatants of several of the cell lines prepared in paragraph C.3 were tested for specific antibody production against types Al and B red blood cells using an agglutination assay. A positive result was defined using V bottom microtiter trays (Falcon Lab) and assuming macroscopic agglutination according to the method of Parker, J. et al, _Transfusion_, 18: 417 (1978) after addition of test solution and centrifugation at 250 x g for 45 seconds. Quantitation was achieved by testing serial doubling dilutions with 3% bovine serum albumin in normal saline  the end point being defined as the limiting dilution.

The results are shown in Table 1.

Table 1:  Frequency and Titer of Hybridomas Secreting
          Human Anti-A Antibody*

| Secreting Line | Number of Wells with Anti-A Activity | Titer ** |
|---|---|---|
| EBV line | | 1: 2,000 |
| SP2/anti A hybrids | | |
| Parents | 19/22 | 1:32 |
| Clones | 0/23 | 0 |
| Triomas SBC-H20/anti-A hybrids | | |
| Parents | 29/31 | 1: 8,192 |
| Clones | 13/21 | 1:16,384 |
| Subclones | 36/53 | 1:32,768 |

*   All antigen specific IgM with kappa light chain
**  Maximum dilution permitting detection of
    agglutination

-28-

The superiority of the SBC-H20 as a fusion partner to obtain a stable antibody secreting line is shown by these results. While fusion of the EBV line with the murine SP2 gave anti-A secreters, the titer was several hundred fold less than the fusion with SBC-H20, and the secretion capability was lost after one transfer. The triomas, on the other hand, maintained (and increased titer) secretion capability after at least two transfers.

The triomas formed by fusion with SBC-H20 were capable of secreting monoclonal anti-A at titers at least four fold higher than the parent EBV transformed lymphocytes themselves through several transfers, and for a period of more than 8 months. The amounts produced are more than 10 µg/ml; comparable to the level of production by a typical murine-murine hybridoma. The quantity produced by the unhybridized EBV transformed cell line E6C6, however, was approximately 10 fold less. The specificity of this immunoglobulin is shown in Table 2. Supernatants were tested using the agglutination assay against 173 donor derived red blood cell preparations which were classified as the groups shown.

Table 2: Reactivity of Human Monoclonal Anti-A
Antibody Against Random Donor Cells

| Positively Testing Cells | Number Tested | % Positive |
|---|---|---|
| A2 | 13 | 100 |
| A1 | 59 | 100 |
| A1B | 4 | 100 |
| A2B | 1 | 100 |
| Aint | 7 | 100 |
| B | 16 | 0 |
| O | 71 | 0 |

A sample of the trioma, designated S-H22, was selected for preservation and deposit.

The antibody may be produced by growing the exemplified hybrid cell lines in a suitable culture medium such as ICM or RPMI (preferably serum free) or *in vivo* in a host such as a nude mouse. If desired, the antibody may be separated from the culture medium or body fluid, as the case may be, by conventional techniques such as ammonium sulfate precipitation, ion exchange chromatography, affinity chromatography, electrophoresis, microfiltration, and ultracentrifugation.

The monoclonal antibody may be used in conventional manual or automated ABO agglutination typing tests to ascertain whether blood samples contain blood type A cells. These tests are based on the ability of the antibody to agglutinate blood type A cells selectively and involve incubating the sample in a container such as a tube or plate well with the antibody and observing the degree of agglutination either visually or spectrometrically. The rate of sedimentation may be enhanced by centrifugation, if desired. Supernatant from the cultures of the exemplified anti-A producing hybrid cells may be used directly in these tests.

Labeled derivatives of the antibody may be made for use in ABO typing via other immunoassay techniques such as radioimmunoassay, fluorescence immunoassay or enzyme immunoassay. The labels that are used in making labeled versions of the antibody include moieties that may be detected directly, such as fluorochromes and radiolabels, as well as moieties, such as enzymes, that must be reacted or derivatized to be detected. Examples of such labels are $^{32}P$, $^{125}I$, $^{3}H$,

$^{14}C$, fluorescein and its derivatives, rhodamine and its derivatives, dansyl, umbelliferone, luciferia, 2,3-dihydrophthalazinediones, horseradish peroxidase, alkaline phosphates, lysozyme, and glucose-6-phosphate dehydrogenase. The antibody may be tagged with such labels by known methods. For instance, coupling agents such as aldehydes, carbodiimides, dimaleimide, imidates, succinimides, bis-diazotized benzadine and the like may be used to tag the antibodies with the above-described fluorescent, chemiluminescent, and enzyme labels.

Blood typing tests using these labeled derivatives will involve incubating a blood sample with the labeled antibody and detecting the presence or absence of labeled immune complexes on the cells in the sample via the label. The details of such procedures are well known in immunoassay art.

The succeeding paragraphs C.8 and C.9 describe the preparation of human antibodies against Varicella Zoster Virus (VZV) for potential diagnostic or therapeutic use. While the disease caused by VZV is commonly trivial (chicken pox or herpes zoster), it can be serious in an immunocompromised host. The human monoclonal antibodies, whose preparation is described below, can be used for therapy in such hosts by parenteral administration to the subject.

C. 8.  **Preparation of Triomas Secreting Antibody Against Varicella Zoster Virus (VZV).**

The triomas secreting anti-VZV were prepared by fusion of the SBC-H20 cell line with lymphocytes isolated from the peripheral blood of a donor with acute varicella infection but who is otherwise normal. The blood lymphocytes were separated using the methods described in paragraph C.3. T cells were removed by rosetting, Saxon (supra), and the isolated B lymphocytes used for fusion herein. The fusion procedure was exactly as described in paragraph C.1 except that IDMEM medium was used instead of RPMI and the cells transferred to microtiter plates at $10^5$ cells/well over irradiated spleen feeder cells in HAT selection medium (as described in paragraph C.3); except that the addition of ouabain is not required as the lymphocytes are untransformed. Hybridoma growth was observed in 37 or 58 seeded wells after 2 weeks and HAT selection was continued for 3 weeks. Monoclonality was verified by sequential limiting dilution cloning, using microtiter plates. Thus, 50% of the hybrids were secreting anti-VZV, as assayed by the method of paragraph C.10.c below.

Several wells were cloned at 0.5 cells per well; clones from two different parent wells showed positive reactivity in the RIA anti-VZV assay of paragraph C.10.c. Some of these were again subcloned, and at least two of the subclones assayed from both parent wells showed anti-VZV IgG levels 10 µg/$10^6$ cells/24 hours as determined by ELISA, and in the viral plaque reduction assay of paragraph C.10.d, showed neutralization at 5 µg/ml of protein. One of these subclones was designated SBC-H21, deposited at ATCC on December 13, 1983, and given ATCC # HB 8463.

C.9.  **Characterization of Mab Secreted**
**by the Trioma.**

Multiple hybrid clones secreting anti-VZV antibodies have been obtained by soft agar and limiting dilution techniques. The viral specific antibody of one of the clones has been further characterized. Several of the clones secrete different antibodies specific for distinct viral epitopes since the clones were derived from multiple hybrid parents and some produce antibody with lambda and others with kappa light chains as determined by enzyme-linked immunoassay. All secreting subclones have produced antigen-specific IgG for greater than eight months after the initial fusion.

C.10. Assays.

The following paragraphs set forth assay procedures useful in the illustrations of the Invention.

C.10.a. Assay for Immunoglobulin Secretion.

Where it was desirable for general or class specific immunoglobulin production to be assessed, immunoglobulin content in the supernatant was determined by enzyme linked immunoassay. Affinity purified, class specific goat anti-human immunoglobulin for example (IgG (Tago, Burlingame, California) is adsorbed onto each well of a flexible flat-bottom microtiter tray (Dynatech Laboratory, Alexandria, Virginia) for 5 hours, and then left overnight at 4°C. After aspiration of the coating each well is incubated in 5% bovine serum albumin (BSA) in phosphate buffered saline (PBS) for an hour at 37°C. After washing with 3x cold PBS and air drying, 25 lambda of hybridoma or trioma supernatant or, as a control, a known amount of purified human IgG is added, and the trays incubated overnight at 4°C. After again washing 3x with cold PBS and air drying, 100 lambda of dilute alkaline phosphatase conjugated goat albumin IgG heavy chain specific antibody (Tago) was added to each well (1:100 dilution) and the plates incubated for 1 hour at 4°C. The plates were again washed and 100 lambda of p-nitrophenyl disodium phosphate (1 mg per ml) in 10% diethylamine buffer pH

0148644

-34-

9.6 added to each well. The plates were incubated 1-2 hours at room temperature. Color changes were read by Dynatech Microplate Reader MR600. The tests were specific for each Ig class over a range of 2 ng per ml to 50 µg per ml.

C.10.b.  Assay for HLA Antigen.

The presence of HLA antigens was assayed by indirect immunofluorescent binding with W6/32 murine monoclonal antibody, which recognizes a framework determinant on all HLA-A, B & C antigens (Brodsky, F.M., et al, Immunological Rev 47:1 (1979)). Briefly, cells were incubated with W6/32 followed by staining with fluorescent goat anti-mouse IgG (Tago) and analysis on an Ortho Cytofluorograph System 50H cell sorter, all by procedures known in the art.

C.10.c.  Radioimmunoassay.

The presence of anti-VZV in the supernatants of the triomas was determined using radioimmunoassay (RIA) by the method of Arvin, A., et al, J Clin Microbiol, 12:367 (1980). Briefly, commercial VZV antigen (Flow Laboratories) was added to each well of a polyvinyl "U"-plate (Dynatech Co., Alexandria, Virginia) and the plate allowed to dry overnight at room temperature. The wells were washed with PBS, filled with PBS containing 20% fetal calf serum, incubated for one hour at 37°, rewashed several times with PBS, and dried. Supernatants to be tested were added to two wells containing antigen and the plates were then incubated 1 hour at 37° and washed 3X with PBS containing 0.05% Tween 20. Goat anti-human IgG, specific for the Fc fraction (Tago, Burlingame, California), labeled with I(125) by a modified chloramine T method, was diluted to contain $2x10^5$ cpm per ml and 250 lambda of this solution was added to each well. The plates were incubated for one hour at 37°, washed with cold PBS containing 0.05% Tween 20, and allowed to dry. The wells were separated by passing a hot wire below the surface of the plate, transferred to a counting vial and counted for one minute in a gamma scintillation counter (Beckman Instruments, Inc., Fullteron, CA). Controls used in each assay include known positive and negative sera and diluent alone. Specific antibody binding was considered to be present at each well for which the ratio of the mean cpm of the two wells containing supernatant and VZV antigen to mean cpm of the two wells containing serum and control antigen was greater than or equal to 2.5.

C.10.d. VZV Viral Neutralization.

VZV antibody was assessed by the plaque reduction multiplicity analysis. The assay is as described by Grose, et al, J Infect Dis, 139:432 (1979) using melanoma and VZV-32 obtained from him. Briefly, 200 lambda of a serial dilution of the partially purified human anti-VZV monoclonal antibody obtained by 45% ammonium sulfate preciptation of a serum free supernatant (HB101, Hana Biologic, Berkeley, CA) or murine monoclonal anti-VZV as a control was added to an equal volume of a stock cell-free VZV-32 to give a final concentration of 500 plaque forming units (pfu) per ml in IDMEM Gibco) without serum and incubated at 37°C for 60 minutes, with intermittent shaking. After incubation, 200 lambda of the virus-antibody mix was inoculated into duplicate wells, in 24-well tissue culture plates (Costar, Cambridge, MA) containing a near confluent layer of melanoma cells which had been grown in IDMEM with 12.5% FCS supplemented with L-glutamine, non-essential amino acids, penicillin and gentamicin. Additionally, eight wells were inoculated with VZV-32 that had been diluted 1:1 (to yield 50 pfu/0.2 ml). The plates were incubated at 32°C with intermittent shaking for 60 minutes. After suctioning off supernatants, the wells were filled with 1 ml of DMEM with 5% FCS and 0.75% carboxymethylcellulose (Sigma, St. Louis, MO). The plates were then incubated at 32°C, 5% $CO_2$ for seven to ten days. Subsequently, the wells were aspirated, and cells fixed and stained with crystal violet in 5% formaldehyde for three hours. After aspiration and overnight drying, plaques were counted with a dissecting microscope.

Samples of the hybrid cell lines HHA1, S-H22, SBC-H20 and SBC-H20 were deposited at the American Type Culture Collection (ATCC), 12301 Parklawn Drive, Rockville, Maryland, U.S.A. The deposit and accession numbers are listed below.

| Cell Line | Deposit Date | Accession No. |
| --- | --- | --- |
| S-H22 | 1 March 1984 | HB8518 |
| HHA1 | 28 March 1984 | HB8534 |
| SBC-H20 | 13 December 1983 | HB8464 |
| SBC-H21 | 13 December 1983 | HB8463 |

These deposits were made under the Budapest Treaty and will be maintained and made available in accordance with the provisions thereof.

Claims

1. An immortalizing, stable, non-secreting murine myeloma-human lymphocyte hybridoma cell line having human characteristics (ie. produces human HLA surface antigen), and the fused products thereof.

2. The hybridoma of Claim 1 which is prepared by fusing murine myeloma cells with human peripheral B lymphocytes derived from a normal donor, selecting the fusion product for stability in immunoglobulin secretion and HLA surface antigen production, treating the selected fusion product with mutagen, and selecting the mutated product for non-secretion of immunoglobulin, but stable production of HLA antigens.

3. The hybridoma of Claim 2 which is further selected for ouabain resistance.

4. The hybridoma SBC-H20, (ATCC HB 8464).

5. A trioma cell line derived from fusion of the hybridoma of Claim 1.

6. The trioma cell line of Claim 5 wherein the hybridoma is fused with human lymphoid cells immunized against a pre-defined determinant.

7. The trioma cell line SBC-H21 (ATCC HB 8463) and progeny thereof.

8. Hybrid cell line S-H22 or HHA1 and progeny thereof.

9. A method for preparing a desired human monoclonal antibody which method comprises fusing lymphoid cells immunized against a predefined determinant with the hybridoma of Claim 1.

10. A method for preparing a desired monoclonal antibody which method comprises culturing the cell line of Claim 7.

11. A method for preparing a desired monoclonal antibody which method comprises culturing the cell line of Claim 8.

12. Mab prepared by the method of Claim 10.

13. Mab prepared by the method of Claim 11.

14. A human monoclonal antibody (Mab) specific for varicella zoster virus antigen and the conjugated products thereof.

15. Anti-varicella zoster virus Mab obtained from trioma SBC-H21 (ATCC HB 8463) and its functional equivalents and the conjugated products thereof.

16. A method of detecting the presence of varicella zoster virus antigen in a sample which method comprises incubating the sample with the Mab of Claim 15 and detecting the presence of the immune complex in the incubation product.

17. A kit for detecting the presence of varicella zoster virus in a sample, which kit comprises:

(a)  the Mab of Claim 15;

(b)  a detecting system for the immune complex between Mab of Claim 15 and varicella zoster virus antigen.

18.  A method of purifying varicella zoster antigen which method comprises reacting a mixture containing said varicella zoster antigen with the Mab of Claim 15.

19.  A human monoclonal antibody having the characteristics of human monolonal antibody S-H22.

20.  Human anti-blood group A monoclonal antibody S-H22 and functional equivalents thereof.

21.  A method of determining whether a human blood sample contains red cells having a type A antigenic determinant comprising incubating the sample with the antibody or functional equivalent of claim 19 and observing whether the reagent agglutinates said cells.

22.  A labeled derivative of the antibody or functional equivalent of claim 19 comprising a conjugate of said antibody or equivalent and a detectable label.

23.  A method of determining whether a human blood sample contains red cells having a type A antigenic determinant comprising incubating the sample with a labeled derivative of claim 22 and observing whethe labeled immune complexes are present on the surface of the cells in the sample.